(19) Europäisches Patentamt
European Patent Office
Office européen des brevets

(11) Veröffentlichungsnummer : **0 036 591**
**B1**

(12) # EUROPÄISCHE PATENTSCHRIFT

(45) Veröffentlichungstag der Patentschrift : **16.11.83**

(51) Int. Cl.[3] : **C 07 C 93/14**, A 61 K 7/13// C07C79/35

(21) Anmeldenummer : **81101901.7**

(22) Anmeldetag : **14.03.81**

(54) **Neue Kupplerkomponenten für Oxidationshaarfarben, deren Herstellung und Verwendung sowie diese enthaltende Haarfärbemittel.**

(30) Priorität : **22.03.80 DE 3011191**

(43) Veröffentlichungstag der Anmeldung : **30.09.81 Patentblatt 81/39**

(45) Bekanntmachung des Hinweises auf die Patenterteilung : **16.11.83 Patentblatt 83/46**

(84) Benannte Vertragsstaaten :
**AT BE CH DE FR GB IT LI LU NL SE**

(56) Entgegenhaltungen :
**EP A 0 011 844**
**DE A 1 719 381**
**DE A 2 737 138**

**Die Akte enthält technische Angaben, die nach dem Eingang der Anmeldung eingereicht wurden und die nicht in dieser Patentschrift enthalten sind.**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Rose, David, Dr.**
**Holbeinweg 7**
**D-4010 Hilden (DE)**
Erfinder : **Möller, Hinrich, Dr.**
**Schumannstrasse 11**
**D-4019 Monheim (DE)**
Erfinder : **Maak, Norbert, Dr.**
**Liebigstrasse 18**
**D-4040 Neus (DE)**

**EP 0 036 591 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

« Neue Kupplerkomponenten für Oxidationshaarfarben, deren Herstellung und Verwendung sowie diese enthaltende Haarfärbemittel »

Gegenstand der Erfindung sind neue Bis-(2,4-diaminophenoxy)-alkanole der allgemeinen Formel

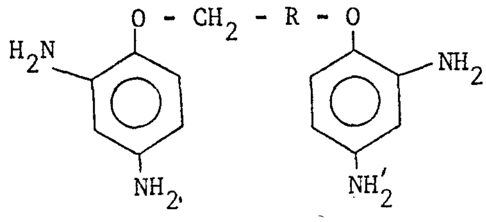

in der R eine der Gruppen

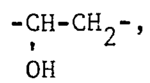 , 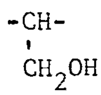 oder 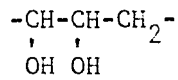

darstellt.

Die Herstellung der neuen Bis-(2,4-diaminophenoxy)-alkanole erfolgt nach bekannten Verfahren der organischen Chemie in mehreren Stufen. Zunächst wird in erster Stufe 2,4-Dinitrophenol mit dem entsprechenden Dibromalkanol zu dem Bis-(2,4-dinitrophenoxy)-alkanol umgesetzt. Dieses wird dann in zweiter Stufe durch katalytische Hydrierung in das gewünschte Bis-(2,4-diaminophenoxy)-alkanol umgewandelt. Die Reaktion verläuft dabei nach folgendem Schema, wobei R die vorgenannte Bedeutung hat :

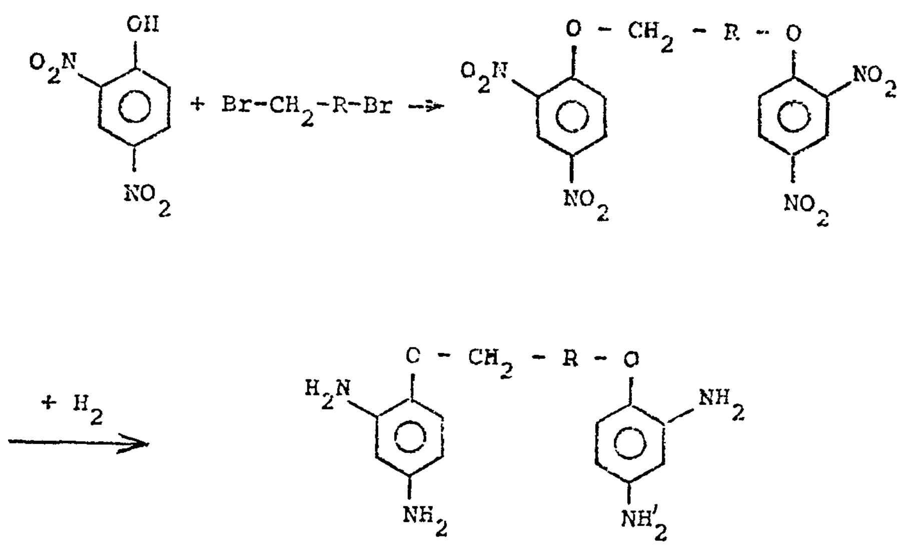

Weitere Gegenstände der Erfindung sind die Verwendung der neuen Bis-(2,4-diaminophenoxy)-alkanole als solcher oder in Gestalt ihrer Salze mit anorganischen oder organischen Säuren als Kupplerkomponenten in Oxidationshaarfarben sowie Haarfärbemittel, die die neuen Bis-(2,4-diaminophenoxy)-alkanole bzw. deren Salze enthalten.

Für das Färben von Haaren spielen die sogenannten Oxidationsfarben, die durch oxidative Kupplung einer Entwicklerkomponente mit einer Kupplerkomponente entstehen, wegen ihrer intensiven Farben und sehr guten Echtheitseigenschaften eine bevorzugte Rolle. Als Entwicklersubstanzen werden üblicherweise Stickstoffbasen wie p-Phenylendiaminderivate, Diaminopyridine, 4-Aminopyrazolon-derivate, heterocyclische Hydrazone eingesetzt. Als sogenannte Kupplerkomponenten werden m-Phenylendiaminderivate, Phenole, Naphthole, Resorcinderivate und Pyrazolone genannt.

Aus der deutschen Patentanmeldung DE-A-2 737 138 sind 2,4-Diaminophenolderivate und Oxidationsfärbemittel für Keratinfasern bekannt, die diese Verbindungen als Kuppler enthalten. Die aus dieser Druckschrift bekannten Verbindungen enthalten nur einen 2,4-Diaminophenoxy-Rest im Molekül und befriedigen noch nicht im Hinblick auf die toxikologischen Eigenschaften.

Aus der deutschen Offenlegungsschrift DE-A 17 19 381 sind Bis-(2-aminophenoxy)-alkane und Bis-(3-aminophenoxy)-alkane als Kuppler für Azofarbstoffe bekannt. Hinweise auf ihre Eignung als Kuppler für Oxidationsfarbstoffe oder für Haarfärbemittel sind dieser Druckschrift nicht zu entnehmen.

Gute Oxidationshaarfarbstoffkomponenten müssen in erster Linie folgende Voraussetzungen erfüllen :

Sie müssen bei der oxidativen Kupplung mit den jeweiligen Entwickler- beziehungsweise Kupplerkomponenten die gewünschten Farbnuancen in ausreichender Intensität ausbilden. Sie müssen ferner ein ausreichendes bis sehr gutes Aufziehvermögen auf menschlichem Haar besitzen und sie sollen darüber hinaus in toxikologischer und dermatologischer Hinsicht unbedenklich sein.

Es bestand daher bei der Suche nach brauchbaren Oxidationshaarfarbstoffen die Aufgabe, geeignete Komponenten aufzufinden, die vorgenannte Voraussetzungen in optimaler Weise erfüllen.

Es wurde nun gefunden, daß man zu Oxidationshaarfarben, die den gestellten Anforderungen in besonders hohem Maße gerecht werden, verlangt, wenn man als Kuppler komponenten Bis-(2,4-diaminophenoxy)-alkanole der allgemeinen Formel

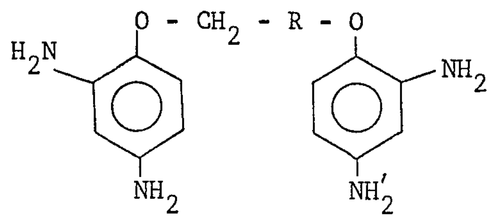

in der R eine der Gruppen

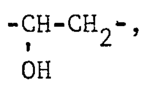, 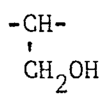 oder 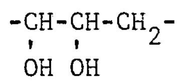,

darstellt, sowie deren Salze mit anorganischen oder organischen Säuren in Kombination mit üblichen Entwicklersubstanzen verwendet.

Haarfärbemittel auf Basis von Oxidationsfarben mit einem Gehalt an Bis-(2,4-diaminophenoxy)-alkanolen der allgemeinen Formel

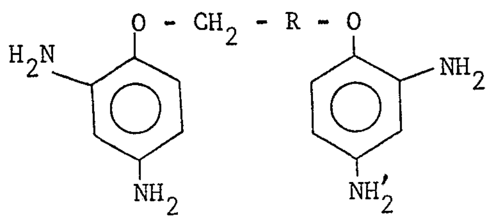

in der R die vorgenannte Bedeutung hat, sowie deren Salzen mit anorganischen oder organischen Säuren als Kupplerkomponenten und den in Oxidationshaarfarben üblichen Entwicklersubstanzen stellen demnach besonders wertvolle Kombinationen auf dem Gebiet der Oxidationshaarfarben dar.

Bei ihrem Einsatz als Kupplerkomponenten liefern die erfindungsgemäßen Verbindungen mit den im allgemeinen für die Oxidationshaarfärbung verwendeten Entwicklersubstanzen sehr intensive, von rotbraun bis schwarzblau reichende Farbtöne und stellen somit eine wesentliche Bereicherung der oxidativen Haarfärbemöglichkeiten dar. Darüber hinaus zeichnen sich die erfindungsgemäßen Bis-(2,4-diaminophenoxy)-alkanole durch sehr gute Echtheitseigenschaften der damit erzielten Färbungen, durch eine gute Löslichkeit in Wasser, eine gute Lagerstabilität und toxikologische sowie dermatologische Unbedenklichkeit aus.

Die erfindungsgemäß als Kupplerkomponenten zu verwendenden Bis-(2,4-diaminophenoxy)-alkanole können entweder als solche oder in Form ihrer Salze mit anorganischen oder organischen Säuren wie zum Beispiel als Chloride, Sulfate, Phosphate, Acetate, Propionate, Lactate, Citrate eingesetzt werden.

Als erfindungsgemäß einzusetzende Kupplerkomponenten sind 1,3-Bis-(2',4'-diaminophenoxy)-2-propanol, 1-Hydroxymethyl-1,2-bis-(2',4'-diaminophenoxy)-ethan und 1,4-Bis-(2',4'-diaminophenoxy)-butan-2,3-diol, sowie deren Salze mit anorganischen oder organischen Säuren zu nennen.

Als Beispiel für in den erfindungsgemäßen Haarfärbemitteln einzusetzende Entwicklerkomponenten sind primäre aromatische Amine mit einer weiteren in p-Stellung befindlichen funktionellen Gruppe wie p-Phenylendiamin, p-Toluylendiamin, p-Aminophenol, N-Methyl-p-phenylendiamin, N,N-Dimethyl-p-phenylendiamin, N,N-Diethyl-2-methyl-p-phenylendiamin, N-Ethyl-N-hydroxyethyl-p-phenylendiamin, Chlorp-phenylendiamin, N,N-Bis-hydroxyethylamino-p-phenylendiamin, Methoxy-p-phenylendiamin, 2-Chlor-, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, andere Verbindungen der genannten Art, die weiterhin eine oder mehrere funktionelle Gruppen wie OH-Gruppen, $NH_2$-Gruppen, NHR-Gruppen, $NR_2$-Gruppen, wobei R einen Alkyl- oder Hydroxyalkylrest mit 1-4 Kohlenstoffatomen darstellt, ferner Diaminopyridinderivate, heterocyclische Hydrazonderivate wie 1-Methyl-pyrrolidon-(2)-hydrazon, 4-Aminopyrazolonderivate wie 4-Amino-1-phenyl-3-carbamoylpyrazolon-5, N-Butyl-N-sulfobutyl-p-phenylendiamin, Tetraaminopyrimidine wie 2,4,5,6-Tetraaminopyrimidin, 4,5-Diamino-2,6-bismethylaminopyrimidin, 2,5-Diamino-4-diethylamino-6-methyl-aminopyrimidin, 2,4,5-Triamino-6-dimethyl-aminopyrimidin, 2,4,5-Triamino-6-piperidino-pyrimidin, 2,4,5-Triamino-6-anilino-pyrimidin, 2,4,5-Triamino-6-morpholino-pyrimidin, 2,4,5-Triamino-6-β-hydroxyethylaminopyrimidin anzuführen.

Die erfindungsgemäßen Kupplerkomponenten liefern neben anderen Farbnuancen mit entsprechenden Entwicklersubstanzen auch dunkelblaue bis schwarzblaue, besonders intensive Haarfärbungen, die sich durch außerordentliche Lichtechtheiten auszeichnen. Sie sind daher auch als Nuancierkomponente zur Erzielung möglichst kräftiger und den natürlichen Haarfarbennuancen weitgehend entsprechender Farbtöne von besonderer Wichtigkeit, da sich bei der Erstellung natürlicher Farbnuancen unter Zuhilfenahme von Blaukupplerkomponenten häufig Schwierigkeiten ergeben.

In den erfindungsgemäßen Haarfärbemitteln werden die Kupplerkomponenten im allgemeinen in etwa molaren Mengen, bezogen auf die verwendeten Entwicklersubstanzen, eingesetzt. Wenn sich auch der molare Einsatz als zweckmäßig erweist, so ist es jedoch nicht nachteilig, wenn die Kupplerkomponente in einem gewissen Überschuß oder Unterschuß zum Einsatz gelangt.

Es ist ferner nicht erforderlich, daß die Entwicklerkomponente und die Kupplersubstanz einheitliche Produkte darstellen, vielmehr können sowohl die Entwicklerkomponente Gemische der erfindungsgemäß zu verwendenden Entwicklerverbindungen als auch die Kupplersubstanz Gemische der erfindungsgemäß einzusetzenden Bis-(2,4-diamino-phenoxy)-alkanole darstellen.

Darüber hinaus können die erfindungsgemäßen Haarfärbemittel gegebenenfalls übliche direktziehende Farbstoffe im Gemisch enthalten, falls dies zur Erzielung gewisser Farbnuancen erforderlich ist.

Die oxidative Kupplung, das heißt die Entwicklung der Färbung, kann grundsätzlich wie bei anderen Oxidationsfarbstoffen auch, durch Luftsauerstoff erfolgen. Zweckmäßigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäßen Haarfärbemittel werden für den Einsatz in entsprechende kosmetische Zubereitungen wie Cremes, Emulsionen, Gele oder auch einfache Lösungen eingearbeitet und unmittelbar vor der Anwendung auf dem Haar mit einem der genannten Oxidationsmittel versetzt. Die Konzentration derartiger färberischer Zubereitungen an Kuppler-Entwicklerkombination beträgt 0,2 bis 5 Gewichtsprozent, vorzugsweise 1 bis 3 Gewichtsprozent. Zur Herstellung von Cremes, Emulsionen oder Gelen werden die Farbstoffkomponenten mit den für derartige Präparationen üblichen weiteren Bestandteilen gemischt. Als solche zusätzlichen Bestandteile sind zum Beispiel Netz- oder Emulgiermittel von anionischen oder nichtionogenen Typ wie Alkylbenzolsulfonate, Fettalkoholsulfate, Alkylsulfonate, Fettsäurealkanolamide, Anlagerungsprodukte von Ethylenoxid an Fettalkohole, Verdickungsmittel wie Methylcellulose, Stärke, höhere Fettalkohole, Paraffinöl, Fettsäuren, ferner Parfümöle und Haarpflegemittel wie Pantothensäure und Cholesterin zu nennen. Die genannten Zusatzstoffe werden dabei in den für diese Zwecke üblichen Mengen eingesetzt, wie zum Beispiel Netz- und Emulgiermittel in Konzentrationen von 0,5 bis 30 Gewichtsprozent und Verdickungsmittel in Konzentrationen von 0,1 bis 25 Gewichtsprozent, jeweils bezogen auf die gesamte Zubereitung.

Die Anwendung der erfindungsgemäßen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8-10 erfolgen. Die Anwendungstemperaturen bewegen sich dabei im Bereich von 15 bis 40 °C. Nach einer Einwirkungsdauer von circa 30 Minuten wird das Haarfärbemittel vom zu färbenden Haar durch Spülen entfernt. Hernach wird das Haar mit einem milden Shampoo nachgewaschen und getrocknet.

Die mit den erfindungsgemäßen Haarfärbemitteln erzielbaren Färbungen, insbesondere auch die Blautöne zeigen unter Einsatz unterschiedlicher Entwickler- und Kupplerkomponenten besonders intensive Farbnuancen. Die erzielten Färbungen haben gute Licht-, Wasch- und Reibechtheitseigenschaften und lassen sich leicht mit Reduktionsmitteln wieder abziehen.

Die nachfolgenden Beispiele sollen den Erfindungsgegenstand näher erläutern, ohne ihn jedoch hierauf zu beschränken.

## Beispiele

Zunächst wird nachstehend die Herstellung der erfindungsgemäßen Bis-(2,4-diaminophenoxy)-alkanole beschrieben.

A) 1,4-Bis-(2',4'-diaminophenoxy)-butan-2,3-dioltetrahydrochlorid

1. Stufe : Herstellung von 1,4-Bis-(2',4'-dinitrophenoxy)-butan-2,3-diol.

19,2 g (0,1 Mol) 2,4-Dinitrophenol (96 %ig) wurden mit 12,4 g (0,05 Mol) 1,4-Dibrombutan-2,3-diol und 6,9 g (0,05 Mol) Kaliumcarbonat in 20 ml Diglykoldimethylether 6 Stunden unter Rückfluß gekocht. Nach dem Abkühlen wurde das Produkt abgesaugt, mit Wasser nachgewaschen und getrocknet. Das erhaltene Zwischenprodukt schmilzt bei 207-210 °C.

2. Stufe : 1,4-Bis-(2',4'-diaminophenoxy)-butan-2,3-diol-tetrahydrochlorid.

4,7 g des in erster Stufe erhaltenen 1,4-Bis-(2',4'-dinitrophenoxy)-butan-2,3-diol wurden in 100 ml Ethanol in Gegenwart von 5 % Palladium auf Kohle katalytisch hydriert. Nach beendeter Wasserstoffaufnahme wurde vom Katalysator abgetrennt, die Lösung mit konzentrierter Salzsäure angesäuert und zur Trockne eingeengt. Die gewünschte Verbindung wurde in Form rotbrauner Kristalle erhalten, die unter Zersetzung bei 170 °C schmolzen.

B) 1,3-Bis-(2',4'-diaminophenoxy)-2-propanol-tetrahydrochlorid-dihydrat

1. Stufe : Herstellung von 1,3-Bis-(2',4'-dinitrophenoxy)-2-propanol.

19,2 g (0,1 Mol) 2,4-Dinitrophenol (96 %ig) wurden mit 13,6 g (0,05 Mol) 1,3-Dibrompropanol und 6,9 g (0,05 Mol) Kaliumcarbonat in 20 ml Diglykoldimethylether 6 Stunden unter Rückfluß gekocht. Nach dem Abkühlen wurde das Produkt abgesaugt, mit Wasser nachgewaschen und getrocknet. Das erhaltene Zwischenprodukt schmilzt bei 152 °C.

2. Stufe : 1,3-Bis-(2',4'-diaminophenoxy)-2-propanoltetrahydrochlorid-dihydrat.

Die Herstellung erfolgte analog den Angaben unter A), 2. Stufe durch katalytische Hydrierung von 1,3-Bis-(2',4'-dinitrophenoxy)-2-propanol. Die gewünschte Verbindung wurde in Form rotbrauner Kristalle erhalten, die unter Zersetzung bei 142 °C schmolzen.

C) 1-Hydroxymethyl-1,2-bis-(2',4'-diaminophenoxy)-ethantetrahydrochlorid

1. Stufe : Herstellung von 1-Hydroxymethyl-1,2-bis-(2',4'-dinitrophenoxy)-ethan.

19,2 g (0,1 Mol) 2,4-Dinitrophenol (96 %ig) wurden mit 13,6 g (0,05 Mol) 2,3-Dibrompropanol und 6,9 g (0,05 Mol) Kaliumcarbonat in 20 ml Dimethylformamid unter Rückfluß 8 Stunden gekocht. Nach dem Abkühlen wurde die Lösung in 1,5 l Wasser gegossen, das ausgefallene Produkt abgesaugt und mit Wasser gewaschen. Das erhaltene Zwischenprodukt schmilzt bei 129 °C unter Zersetzung.

2. Stufe : 1-Hydroxymethyl-1,2-bis-(2',4'-diaminophenoxy)-ethan-tetrahydrochlorid.

Die Herstellung erfolgte analog den Angaben unter A), 2. Stufe durch katalytische Hydrierung von 1-Hydroxymethyl-2,3-bis-(2',4'-dinitrophenoxy)-ethan. Die gewünschte Verbindung wurde in Form brauner Kristalle erhalten. IR-Spektrum (KBr) $cm^{-1}$ : 1 630, 1 560, 1 500, 1 400, 1 335, 1 285, 1 230, 1 130, 1 040, 940, 875, 820.

Die vorgenannten, in ihrer Herstellung beschriebenen Bis-(2,4-diaminophenoxy)-alkanole A-C wurden als Kupplerkomponenten in den folgenden Beispielen eingesetzt :
Als Entwicklerkomponenten dienten folgende Substanzen :

E 1 : p-Toluylendiamin
E 2 : 2,4,5,6-Tetraaminopyrimidin
E 3 : p-Phenylendiamin
E 4 : N,N-Dimethyl-p-phenylendiamin
E 5 : 2-Chlor-p-phenylendiamin
E 6 : 2,5-Diaminoanisol
E 7 : N,N-Bis-(β-hydroxyethyl)-amino-p-phenylendiamin
E 8 : p-Aminophenol
E 9 : N-Methyl-p-phenylendiamin
E 10 : N-Ethyl-N-β-hydroxyethyl-p-phenylendiamin
E 11 : N-Methylamino-4,5,6-triaminopyrimidin.

Die erfindungsgemäßen Haarfärbemittel wurden in Form einer Cremeemulsion eingesetzt. Dabei

wurden in eine Emulsion aus

10 Gewichtsteilen Fettalkoholen der Kettenlänge $C_{12}$-$C_{18}$,
10 Gewichtsteilen Fettalkoholsulfat (Natriumsalz) Kettenlänge $C_{12}$-$C_{18}$ und
75 Gewichtsteilen Wasser

jeweils 0,01 Mol der in der nachstehenden Tabelle aufgeführten Entwicklersubstanzen und Kupplersubstanzen eingearbeitet. Danach wurde der pH-Wert der Emulsion mittels Ammoniak auf 9,5 eingestellt und die Emulsion mit Wasser auf 100 Gewichtsteile aufgefüllt. Die oxidative Kupplung wurde mit 1 %iger Wasserstoffperoxidlösung als Oxidationsmittel durchgeführt, wobei zu 100 Gewichtsteilen der Emulsion 10 Gewichtsteile Wasserstoffperoxidlösung gegeben wurden. Die jeweilige Färbecreme mit Zusatz von Oxidationsmitteln wurde auf zu 90 % ergrautes, nicht besonders vorbehandeltes Menschenhaar aufgetragen und dort 30 Minuten belassen. Nach Beendigung des Färbeprozesses wurde das Haar mit einem üblichen Haarwaschmittel ausgewaschen und anschließend getrocknet. Die dabei erhaltenen Färbungen sind nachstehender Tabelle 1 zu entnehmen.

Tabelle 1

| Beispiel | Entwickler | Kuppler | Erhaltener Farbton mit 1 %iger $H_2O_2$-Lösung |
|---|---|---|---|
| 1 | E 1 | A | schwarzblau |
| 2 | E 2 | A | dunkelblau |
| 3 | E 1 | B | schwarzblau |
| 4 | E 2 | B | dunkelblau |
| 5 | E 1 | C | dunkeltürkis |
| 6 | E 2 | C | blaugrau |
| 7 | E 3 | C | blaugrau |
| 8 | E 4 | C | dunkelblau |
| 9 | E 5 | C | dunkelviolett |
| 10 | E 6 | C | blaugrau |
| 11 | E 7 | C | dunkelblau |
| 12 | E 8 | C | rotbraun |
| 13 | E 9 | C | blaugrau |
| 14 | E 10 | C | blaugrau |
| 15 | E 11 | C | dunkelgrün |

**Ansprüche** (für die Vertragsstaaten : BE, CH, DE, FR, GB, IT, LI, LU, NL, SE)

1. Bis-(2,4-diaminophenoxy)-alkanole der allgemeinen Formel

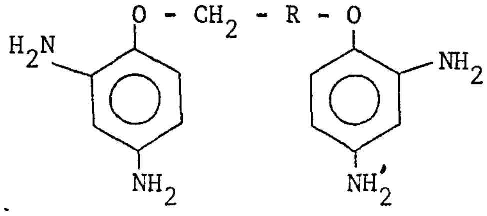

in der R eine der Gruppen

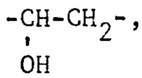, 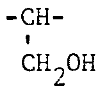 oder 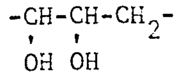

darstellt.

2. Verfahren zur Herstellung der Bis-(2,4-diaminophenoxy)-alkanole gemäß Anspruch 1 dadurch, daß man in erster Stufe 2,4-Dinitrophenol mit einem Dibromalkanol der Formel Br—$CH_2$—R—Br, in der R die vorgenannte Bedeutung hat, nach bekannter Methode umsetzt und danach in zweiter Stufe das erhaltene Bis-(2,4-dinitrophenoxy)-alkanol in bekannter Weise zum Bis-(2,4-diaminophenoxy)-alkanol katalytisch hydriert.

3. Verwendung von Bis-(2,4-diaminophenoxy)-alkanolen gemäß Anspruch 1 und 2, sowie deren Salzen mit anorganischen oder organischen Säuren als Kupplerkomponenten in Oxidationshaarfarbstoffen.

4. Haarfärbemittel auf Basis von Oxidationsfarbstoffen mit einem Gehalt an Bis-(2,4-diaminophenoxy)-alkanolen nach Anspruch 1-3, sowie deren Salzen mit anorganischen oder organischen Säuren als Kupplerkomponenten und den in Oxidationshaarfarben üblichen Entwicklersubstanzen.

5. Haarfärbemittel nach Anspruch 4, gekennzeichnet durch einen Gehalt an Entwickler-Kuppler-Kombination in einer Menge von 0,2 bis 5 Gewichtsprozent, vorzugsweise 1 bis 3 Gewichtsprozent, bezogen auf das gesamte Haarfärbemittel.

**Ansprüche** (für den Vertragsstaat AT)

1. Verfahren zur Herstellung von Bis-(2,4-diaminophenoxy)-alkanolen der allgemeinen Formel I

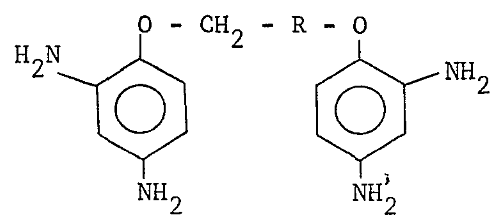

in der R eine der Gruppen

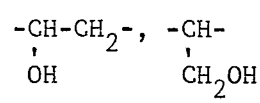

oder

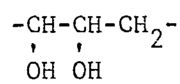

dadurch gekennzeichnet, daß man in erster Stufe 2,4-Dinitrophenol mit einem Dibromalkanol der Formel Br—$CH_2$—R—Br, in der R die vorgenannte Bedeutung hat, nach bekannter Methode umsetzt und danach in zweiter Stufe das erhaltene Bis-(2,4-dinitrophenoxy)-alkanol in bekannter Weise zum Bis-(2,4-diaminophenoxy)-alkanol katalytisch hydriert.

2. Verwendung von Bis-(2,4-diaminophenoxy)-alkanolen der allgemeinen Formel gemäß Anspruch 1 sowie deren Salzen mit anorganischen oder organischen Säuren als Kupplerkomponenten in Oxidationshaarfarbstoffen.

3. Haarfärbemittel auf Basis von Oxidationsfarbstoffen mit einem Gehalt an Bis-(2,4-diaminophenoxy)-alkanolen der allgemeinen Formel I gemäß Anspruch 1 sowie deren Salzen mit anorganischen oder organischen Säuren als Kupplerkomponenten und den in Oxidationshaarfarben üblichen Entwicklersubstanzen.

4. Haarfärbemittel nach Anspruch 3, gekennzeichnet durch einen Gehalt an Entwickler-Kuppler-Kombination in einer Menge von 0,2 bis 5 Gew.-%, vorzugsweise 1-3 Gew.-%, bezogen auf das gesamte Haarfärbemittel.

**Claims** (for the Contracting States : BE, CH, DE, FR, GB, IT, LI, LU, NL, SE)

1. Bis-(2,4-diaminophenoxy)-alkanols corresponding to the following general formula

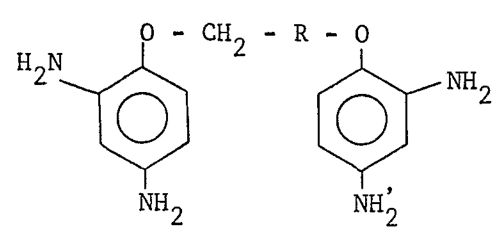

in which R represents one of the following groups

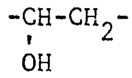, 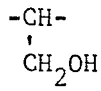 or 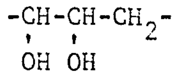

2. A process for producing the bis-(2,4-diaminophenoxy)-alkanols claimed in Claim 1, characterised in that, in a first stage 2,4-dinitrophenol is reacted in known manner with a dibromoalkanol corresponding to the formula $Br—CH_2—R—Br$, in which R is as defined above, and then in a second stage the bis-(2,4-dinitrophenoxy)-alkanol obtained is catalytically hydrogenated in known manner to form bis-(2,4-diaminophenoxy)-alkanol.

3. The use of the bis-(2,4-diaminophenoxy)-alkanols claimed in Claims 1 and 2 and their salts with inorganic or organic acids as coupler components in oxidation hair dyes.

4. Hair dyes based on oxidation dyes containing the bis-(2,4-diaminophenoxy)-alkanols claimed in Claims 1 to 3 and their salts with inorganic or organic acids as coupler components and the developer substances commonly used in oxidation hair dyes.

5. Hair dyes as claimed in Claim 4, characterised by a content of from 0.2 to 5 % by weight and preferably from 1 to 3 % by weight, based on the hair dye as a whole, of developer-coupler combination.

**Claims** (for the Contracting State AT)

1. A process for the production of bis-(2,4-diaminophenoxy)-alkanols corresponding to the following general formula

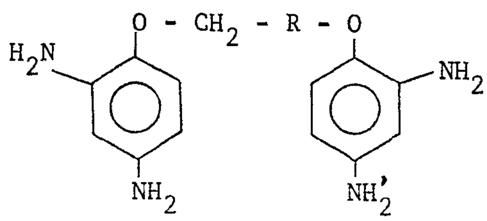

in which R represents one of the following groups

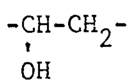, 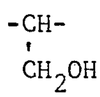 or 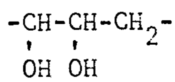

characterized in that, in a first stage, 2,4-dinitrophenol is reacted with a dibromoalkanol corresponding to the formula $Br—CH_2—R—Br$, in which R is as defined above, in known manner and then in a second stage the bis-(2,4-dinitrophenoxy)-alkanol obtained is catalytically hydrogenated in known manner to form bis-(2,4-diaminophenoxy)-alkanol.

2. The use of bis-(2,4-diaminophenoxy)-alkanols corresponding to the general formula in Claim 1 and their salts with inorganic or organic acids as coupler components in oxidation hair dyes.

3. Hair dyes based on oxidation dyes containing bis-(2,4-diaminophenoxy)-alkanols corresponding to general formula I in Claim 1 and their salts with inorganic or organic acids as coupler components and the developer substances commonly used in oxidation hair dyes.

4. Hair dyes as claimed in Claim 3, characterized by a content of from 0.2 to 5 % by weight and preferably from 1 to 3 % by weight, based on the hair dye as a whole, of developer-coupler combination.

**Revendications** (pour les Etats Contractants : BE, CH, DE, FR, GB, IT, LI, LU, NL, SE)

1. Bis-(2,4-diaminophénoxy)-alcanols de formule générale

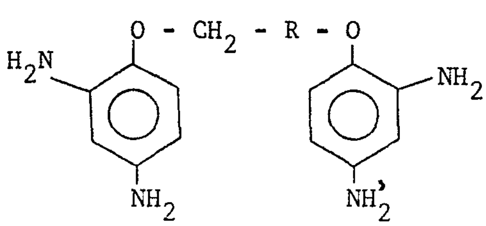

dans laquelle R représente un des groupes

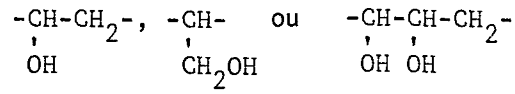

2. Procédé de préparation de bis-(2,4-diaminophénoxy)-alcanols selon la revendication 1, caractérisé en ce que dans un premier stade on fait réagir par une méthode connue du 2,4-dinitrophénol avec un dibromoalcanol de formule Br—$CH_2$—R—Br dans laquelle R a la signification citée plus haut, et en ce qu'ensuite dans un second stade on hydrogène catalytiquement de manière connue le bis-(2,4-dinitrophénoxy)-alcanol obtenu en bis-(2,4-diaminophénoxy)-alcanol.

3. Utilisation de bis-(2,4-diaminophénoxy)-alcanols selon les revendications 1 et 2 ainsi que de leurs sels avec des acides minéraux ou organiques comme composants de couplage dans des colorants d'oxydation pour cheveux.

4. Agents de teinture pour cheveux à base de colorants d'oxydation ayant une teneur en bis-(2,4-diaminophénoxy)-alcanols selon les revendications 1 à 3 de même qu'en leurs sels avec des acides minéraux ou organiques, en tant que composants de couplage et de substances de développement en usage dans la teinture oxydante des cheveux.

5. Agents de teinture pour cheveux selon la revendication 4, caractérisés par une teneur en une combinaison agent de développement-coupleur en une quantité de 0,2 à 5 % en poids, de préférence de 1 à 3 % en poids par rapport à la totalité de l'agent de teinture pour cheveux.

**Revendications** (pour l'Etat Contractant AT)

1. Procédé de préparation de bis-(2,4-diaminophénoxy)-alcanols de formule générale

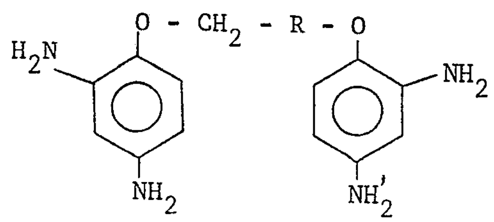

dans laquelle R représente un des groupes

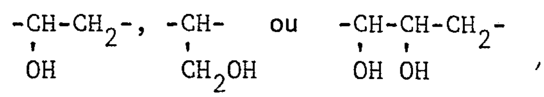

caractérisé en ce que dans un premier stade on fait réagir par une méthode connue du 2,4-dinitrophénol avec un dibromoalcanol de formule Br—$CH_2$—R—Br dans laquelle R a la signification citée plus haut, et en ce qu'ensuite dans un second stade on hydrogène catalytiquement de manière connue le bis-(2,4-dinitrophénoxy)-alcanol obtenu en bis-(2,4-diaminophénoxy)-alcanol.

2. Utilisation de bis-(2,4-diaminophénoxy)-alcanols selon la revendication 1 ainsi que de leurs sels avec des acides minéraux ou organiques comme composants de couplage dans des colorants d'oxydation pour cheveux.

3. Agents de teinture pour cheveux à base de colorants d'oxydation ayant une teneur en bis-(2,4-diaminophénoxy)-alcanols de formule générale I selon la revendication 1 de même qu'en leurs sels avec des acides minéraux ou organiques, en tant que composants de couplage et de substances de développement en usage dans la teinture oxydante des cheveux.

4. Agents de teinture pour cheveux selon la revendication 3, caractérisés par une teneur en une combinaison agent de développement-coupleur en une quantité de 0,2 à 5 % en poids, de préférence de 1 à 3 % en poids par rapport à la totalité de l'agent de teinture pour cheveux.